(19) Europäisches Patentamt
European Patent Office
Office européen des brevets

(11) **EP 2 723 196 B1**

(12) ## EUROPEAN PATENT SPECIFICATION

(45) Date of publication and mention of the grant of the patent:
**03.08.2016 Bulletin 2016/31**

(51) Int Cl.:
*A61K 36/899* *(2006.01)*    *C12P 21/06* *(2006.01)*
*A61P 29/00* *(2006.01)*    *A61P 35/00* *(2006.01)*

(21) Application number: **12729113.6**

(22) Date of filing: **21.06.2012**

(86) International application number:
**PCT/EP2012/061935**

(87) International publication number:
**WO 2012/175594 (27.12.2012 Gazette 2012/52)**

(54) **WHEAT GERM EXTRACT OBTAINED BY PROTEASE HYDROLYSIS AND MEDICAL USE THEREOF**

WEIZENKEIMEXTRAKT GEWONNEN DURCH PROTEASE HYDROLYSE, UND DESSEN MEDIZINISCHE VERWENDUNG

EXTRAIT DE GERME DE BLÉ OBTENU PAR UNE HYDROLYSE À L'AIDE DE PROTÉASE, ET SON UTILISATION MÉDICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2011 GB 201110746**

(43) Date of publication of application:
**30.04.2014 Bulletin 2014/18**

(73) Proprietor: **Biropharma UK Ltd**
**London, Greater London SW7 3DQ (GB)**

(72) Inventors:
• **NAGYPALNE DERI, Klara**
**H-6400 Kiskunhalas (HU)**
• **NAGYPAL, Laszlo**
**H-6400 Kiskunhalas (HU)**

(74) Representative: **Hart, Deborah Mary**
**Kilburn & Strode LLP**
**20 Red Lion Street**
**London**
**WC1R 4PJ (GB)**

(56) References cited:
**WO-A1-99/08694    WO-A1-2004/014406
FR-A1- 2 912 313**

• **HIDVEGI M ET AL: "Effect of Avemar and Avemar + vitamin C on tumor growth and metastasis in experimental animals", ANTICANCER RESEARCH, INTERNATIONAL INSTITUTE OF ANTICANCER RESEARCH, GR, vol. 18, no. 4A, 1 July 1998 (1998-07-01), pages 2353-2358, XP009020669, ISSN: 0250-7005**

Remarks:
The file contains technical information submitted after the application was filed and not included in this specification

EP 2 723 196 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The invention relates to a dried enzymically hydrolyzed plant material, which is capable of immunmodulation, immunoregulation, reducing inflammation and reducing tumor progression. The material can be used in the manufacture of pharmaceutical compositions, dietary supplements, dietary foods for medical purposes, nutraceuticals, in feed materials, and in supplementary feeds. The process for producing the material is also described, as are its use in the modulation, and regulation of immune reactions, reducing inflammation and/or tumor progression. The material can also be used in treatment of several inflammatory disorders.

The composition of the invention can be produced by the enzymic hydrolysis of wheat germ in the presence of pancrease powders (pancreatis pulvis) in a watery medium followed by enzyme inactivation, separation, cleaning, evaporation, and drying of the liquid hydrolysate.

Biologically active agents produced by bio-technological methods from basic materials of an actual origin are becoming increasingly important. Preparations which can prevent pathological changes or can act as adjuvants in therapy to decrease the progression of pathological changes and improve the quality of life of patients are particularly important.

The present invention provides a material derived from wheat germ which can significantly decrease the symptoms of inflammation, in particular in the digestive track. In addition, the material is useful in the treatment of inflammatory diseases, such as rheumatoid arthritis, as well as in the treatment of cancer. The material can also be used as a dietary supplement, a dietary food for medical purposes, and a supplementary feed.

WO 99/08694 describes a material obtained by the fermentation of wheat germ with Baker's yeast (*Saccharomyces cerevisiae*) which was found to have an immunostimulatory and metastasis inhibiting effect. The material contains 0.12-0.52mg/g dry material 2,6,-dimethoxy-p-benzoquinone (2.6-DMBQ). This material, known commercially as Avemar™ pulvis (a fermented wheat germ extract) has also been used as an anti-inflammatory agent for preventing or treating or alleviating inflammatory conditions in particular rheumatoid arthritis as described in WO 2004/014406. WO 2004/014146 also describes the use of this material as a dietary supplement for animals. It provides quicker gain in body weight as well as improving resistance to infectious diseases.

The present invention relates to a new material derived from wheat germ which has been found to have similar biological effects to Avemar™ pulvis. In particular, this material has been shown to be useful in the treatment of inflammatory bowel diseases, as an anti-inflammatory agent, and in the treatment of cancer.

Thus in the first aspect the present invention relates to a process for producing a dried hydrolysed wheatgerm extract comprising

> hydrolysing wheat germ with a protease enzyme in an aqueous medium, inactivating the enzyme;
> separating the residual wheat germ substrate from the hydrolysed liquid; and evaporating and/or drying the hydrolysed liquid.

The hydrolysed dried plant material of the invention is produced from wheat germ. The wheat germ is separated from wheat (*Tricticum vulgare*) during the industrial milling process. The wheat germ consists of characteristic yellowish coloured round or oval particles about 0.8 - 2mm in diameter and about 0.1 - 0.25mm thick.

Preferably the wheat germ is hydrolysed with pancrease powder. Pancrease powder (pancreatis pulvis) is prepared from the fresh or frozen pancreas of mammals. The pancrease powder has proteolytic, lipolitic and amylolytic activities. Pancrease powder is available in Ph. Eur (USP) quality with a standardized enzyme activity. The wheat germ material is hydrolysed with a pancrease powder with a standardized enzyme activity. In the present invention reaction conditions which favour the protease activity are used. This generates a range of a degree of hydrolysis (DH%) in the protein fraction in the material. The DH% is prefereably between 10-50% as determined by TNBS method (Adler Nissen).

The protease activity can vary between the different formulae of pancrease powder available. The ratio of pancrease to substrate (wheat germ) (PA/S ratio) depends upon the proteolytic activity of the powder used. For a standard pancrease powder with 1.5Ph.Eur units/milligrams (approximately 100USP units/ mg) proteolytic activity, the PA/S ratio should be set between 1:750 - 1:1250, preferably 1:1000. Table 1 below shows the ideal PA/S ratio to be used depending on the proteolytic activity of the pancrease powder.

| Proteolytic activity of pancrease powder | | PA/S ratio min. | PA/S ratio max. | PA/S ratio middle |
|---|---|---|---|---|
| *Ph.Eur. units/mg* | *≈ USP units/mg* | | | |
| 1.0 | 67 | 1:500 | 1:833 | 1:667 |
| 1.5 | 100 | 1:750 | 1:1250 | 1:1000 |
| 1.9 | 125 | 1:938 | 1:1563 | 1:1250 |

(continued)

| Proteolytic activity of pancrease powder | | PA/S ratio min. | PA/S ratio max. | PA/S ratio middle |
|---|---|---|---|---|
| Ph.Eur. units/mg | ≈ USP units/mg | | | |
| 2.3 | 150 | 1:1313 | 1:1875 | 1:1500 |
| 3.0 | 200 | 1:1500 | 1:2500 | 1:2000 |

Hydrolysis of the plant material (wheatgerm) is carried out at pH 6.5 to 9.5 preferably about pH 8. This value can be altered using standard techniques to the person skilled in the art such as by using a potassium hydroxide or sodium hydroxide solution. The hydrolysis is carried at about 40 to 60°C, preferably about 50°C. Hydrolysis occurs for 1 to 8 hours, for example 60 to 200 minutes, preferably about 100 minutes. The weight ratio of the wheat germ to water is about 1:6 - 1:11, preferably about 1:8.5. The reaction is usually carried out with continuous stirring. Following the enzymic hydrolysis, the enzymes present are inactivated. Methods of inactivation are well known to the person skilled in the art. For example, the reaction mixture can be heated to 88-98°C, preferably 90°C and maintained at this temperature for a period of time, for example 5 to 20 minutes.

After the enzyme has been inactivated, the hydrolyzed liquid is separated from the residual wheat germ substrate using a suitable method. For example, the liquid can be centrifuged using a spiral decanter-centrifuge or other type of centrifuge on the industrial scale. The hydrolyzed liquid supernatant is then further treated to remove other small particles, by using, for example, a separator, and/or filter press, or other filtering equipment so that a clear liquid is obtained.

The clear liquid is then evaporated, for example by using a vacuum evaporator set at 40 to 50°C. The degree of evaporation should be 10 to 30 % Brix, preferably about 20 Brix%. The concentrated liquid obtained following the evaporation process is further dried, for example by a spray drier. The degree of hydrolysis (DH) in the protein fraction of the hydro lysed, dried plant material obtained by the method is 10-50% as determined by the trinitrobenzene sulphonic acid (TNBS) method (Adler-Nissen).

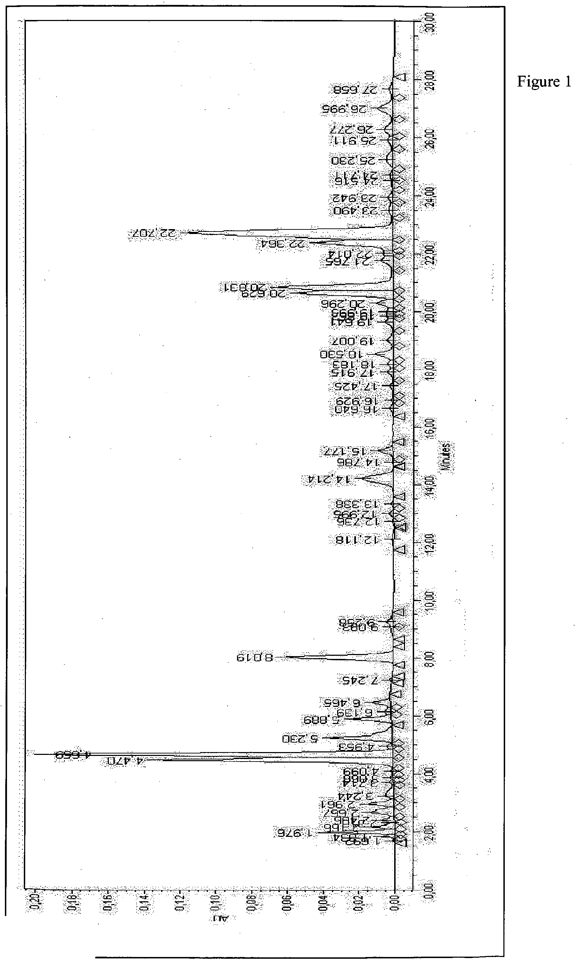

The material of the invention has been analysed by HPLC as described in the following examples. Thus in one embodiment the material has an HPLC chromatogram substantially corresponding to that shown in Figure 1. Analysis of the 2.6-DMBQ content of the material shows that is typically contains less than 0.05 mg/g dry matter 2.6-DMBQ. Therefore it has a different composition to the fermented wheat germ material described in WO 99/08694.

The material of the invention can be used to treat a variety of conditions including inflammatory diseases and to inhibit or prevent tumour progression, such as in cancer.

Thus in a further aspect the invention provides the material of the invention for use in medicine. In particular the material can be used to treat and/or prevent inflammatory conditions and cancer.

As used herein the term "inflammatory diseases" include arthritis, specifically rheumatoid arthritis, reactive arthritis and bacterial arthritis; inflammatory bowel disease, and autoimmune conditions.

As used herein "inflammatory bowel disease" (IBD) relates to inflammatory conditions of the colon and small intestine. This includes Crohn's disease, ulcerative colitis, Collagenous colitis, Lymphocytic colitis, Ischaemic colitis, Diversion colitis, Behcet's disease and Indeterminate colitis. Inflammatory bowel diseases (IBD) are becoming increasingly common. In the case of some types of IBD the inflammatory reaction is localised only on the mucosa (e.g. ulcerative colitis), but in the case of other types of IBD, e.g. in Crohn's disease inflammation occurs in all layers of the bowel wall from the mucosa to the serosa (transmuralic). During the inflammatory process neutrophils and endothelial cells start to produce remarkable quantities of inflammatory-causing cytokines (IL-1, IL-6, TNF-alpha). The main aim of medicinal treatments used for the inflammatory bowel diseases is to ease symptoms of the disease, to suppress the inflammatory process, and prevent the complications (e.g. abscess, fistula). The main groups of medicines used in the treatment of IBD are salicilyic-acid-derives (5 -ASA; SASP), corticosteroids, immunosupressors, antibiotics, and biologically active agents (infliximab). A

In another aspect the invention provides a pharmaceutical composition comprising the material of the invention. The pharmaceutical composition may also contain one or more excipients, diluents or carriers.

The compositions of the invention may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

Pharmaceutical formulations adapted for topical administration may be formulated as ointments, creams, suspensions, lotions, powders, solutions, pastes, gels, sprays, aerosols or oils.

Pharmaceutical formulations adapted for rectal administration may be presented as suppositories or enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists which may be generated by means of various types of metered dose pressurised aerosols, nebulizers or insufflators.

Pharmaceutical formulations adapted for parenteral administration include aqueous and nonaqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the formulations may also include other agents conventional in the art having regard to the type of formulation in question, for example those suitable for oral administration may include flavouring agents.

Preferred unit dosage formulations are those containing a daily dose or sub-dose, or an appropriate fraction thereof, of an active ingredient. Suitable daily dosages are between 0.001- 100g, preferably 0.01-50g, more preferably 0.1 -40g per kg body weight.

In another aspect the disclosure describes a method of treating and/or preventing an inflammatory condition or cancer comprising administering the dried hydrolysed wheatgerm extract or the pharmaceutical composition of the invention to a subject in need thereof.

In a further aspect the invention provides a food or feed comprising the dried hydrolysed wheatgerm extract of the invention. As used herein "food" refers to foodstuff eaten by humans while "feed" refers to feed material given to animals, particularly domesticated animals. The food or feed can contain 0.1-10% by weight, preferably 1-5% by weight the dried plant material of the invention. In addition a dietary supplement, a dietary food for medical purposes (for supplementing medical treatments) for humans and a supplementary feed for animals comprising the dried hydrolysed wheat germ extract of the invention is also provided. The supplements can contain 10-98 %, preferably 50-90 % by weight the dried hydrolysed wheat germ extract of the invention. The hydrolysed dried extract can be added to the food or feed directly or premixed with other vitamins, minerals and known dietary or feed supplements.

As used herein "animals" comprises farm animals such as cattle, sheep, horses, pigs, poultry, rabbits, fish; pets including dogs,cats and other domestic animals; and zoo animals.

The invention will now be described in the following examples with reference to the following figures:

Figure 1 shows the characteristic HPLC chromatogram of the PABD powder.

Figure 2 shows the electrophoregram of PABD powder and densitometric evaluation. Figure 2a shows the proteins separated from PABD powder in 15 % polyacrylamide-gel on the basis of their molecular weight. The protein fractions were marked by colloid-painting. Figure 2b shows the electrophoregram evaluated by a videodensitometer. Figure 2c shows the distribution of protein fractions of PABD powder as compared with mixture of known proteins

Figure 3 shows the effect of PABD powder on body weight change induced by TNBS.

Figure 4 shows the effect of PABD powder on extension of mucous membrane lesions.

Figure 5 shows the effect of PABD powder on seriousness of inflammation induced by TNBS.

Figure 6 shows the effect of PABD powder on TNF-alpha level induced by TNBS (pg/mg protein).

Figure 7 shows the effect of PABD powder on MPO activity induced by TNBS (mU/mg protein).

**Example 1:**

*Laboratory scale preparation of plant material obtained by hydrolysis of wheat germ with pancrease powder.*

200 g fresh, food quality wheat germ was mixed in 1700 ml drinking water The temperature of the suspension was increased to 50°C, and pH value was adjusted to 8.0 ± 0.1 using 22 m/m % KOH solution. 0.2 g pancrease powder with 1.5 Ph.Eur units/mg proteolytic activity was dissolved in 2 ml drinking water and added to the suspension. The enzymic reaction was carried out at 50°C ± 2°C, pH 8.0 ± 0.1 under continually stirring for 100 min.

The enzyme was inactivated by heating to 90°C and keeping the suspension at 90°C ± 2°C for 10 min. After the end of hydrolysis and inactivation the suspension was centrifuged for 20 min at 3600 rpm. The liquid phase was evaporated until 21 Brix with a Rotadest vacuum evaporator. A yellowish colour, lightly foaming concentrate was obtained: the concentrated hydrolysed material was dried by a lab-spray drier. The degree of hydrolysis of the protein fraction of obtained plant hydrolysate powder (PABD powder) was DH = 21% measured by TNBS method (Adler-Nissen).

**Example 2:**

*Plant scale production of plant material obtained by hydrolysis of wheat germ with pancrease powder.*

200 kg fresh, food quality wheat germ was mixed in 1700 L drinking water (filtered to sterile). The suspension was heated by indirect heating until a temperature of 50°C was reached. The pH value was adjusted using 22 m/m% KOH solution to pH on 8.0 ± 0.1. 200g pancrease powder with 1.5 Ph.Eur units/mg proteolytic activity and Ph.Eur quality was dissolved in 1500 ml drinking water and added to the suspension.

Hydrolysis was executed under continually stirring at 50°C ± 1°C for 100 minutes at pH = 8.0 ± 0.2. The enzyme was inactivated by heating the suspension to 90°C and maintaining this temperature (90°C ± 1°C) for 15 min. After this, the reaction mixture was cooled to 70°C and the liquid hydrolysate phase was separated from the residual wheat germ particles by a centrifugal decanter. The liquid phase containing wheatgerm extract was cleared by separators, to obtain a yellowish coloured, clear liquid. This liquid was evaporated by a vacuum-evaporator at 42-45°C until 20 Brix%. A viscosous, yellowish coloured material with a lightly foam forming property was obtained. The concentrated hydrolysate was dried by a high revolution (16 000 rpm) disc spray-drier. The degree of hydrolysis in the protein fraction of the obtained hydrolysed, dried plant material (PABD powder) was DH = 20 %. (Adler-Nissen method).

*Characterization of the hydrolysed, dried plant material according to the invention*

The hydrolysed, dried plant material (PABD powder) according to the invention is a fine powder with a yellowish colour. It has an acceptable taste. In order to characterise the powder the Degree of hydrolysis of the protein fraction was determined according to the method of Adler-Nissen. This provides the ratio of the number of free amino-groups compared to the total number of amino-groups. This DH % value measures the degree of hydrolysis.

*Short description of DH % determination*

The degree of hydrolysis was determined by the TNBS method (Adler-Nissen 1979. J. Agric. Food Chem 27, (6) pp. 1256-1262). This method is based on the chemical reaction between the primary amino-groups of proteins and trinitrobenzene- sulphonic acid (TNBS) under mild alkaline conditions. The compound formed during this reaction is yellow coloured, and the concentration can be measured spectrophotometrically at 340 nm. 1 % SDS solutions containing 2 mmol, 4 mmol, 6 mmol, 8 mmol leucin were used for calibration.

To detemine the total number of amino-acid groups samples were hydrolysed in the presence of 6N hydrochloric acid in $N_2$ atmosphere at 105°C for 25 hours, and after this they were evaporated. Untreated and hydrolysed samples were dissolved in 1 % SDS and examined. For the TNBS test hydrolysed samples, untreated samples and leucin standards were reacted in presence of phosphorate-buffer at pH 8.2 with the TNBS reagent for 60 min. at 50 °C. The reaction was stopped using 0.1 N hydrochloric acid.

The absorbance of the samples at 340nm was measured using a spectrophotometer. A calibration was obtained and the number of free amino-acid groups in the hydrolysed and non-hydrolysed samples was determined on the basis of absorbance expressed in mmol leucin.

leucin.

$$DH \% = (\text{number of free amino-acid groups} / \text{total amino-acid groups}) \times 100$$

The degree of hydrolysis the protein fraction of hydrolysed and dried plant material according to invention is DH: 10-50 %.

**2,6-dimethoxy-p-benzoquinone (2,6 DMBQ) content of the new material (PABD powder)**

_Method:_

**Instruments and chromatographic conditions**

| | |
|---|---|
| **Instrument** | Waters 2696 Separation Module, Alliance |
| | Waters 2996 Photodiode Array Detector |
| | Empower Pro Software, Waters |
| **Column** | Kromasil 100 C18 5$\mu$m 15 x 0.46 (mm) |
| **Elution** | Isocratic |
| | 0,7 ml/min |
| **Detection** | 290 nm |
| **Mobile phase** | - $KH_2PO_4$ - 25 mM/1. <br> - pH 4,8 <br> - 30 % acetonitrile (v/v %) |

**2,6-Dimethoxy-p-benzoquinone** standard used was chromatographical quality.

**Sample preparation**

0.5 g of sample was dissolved in 50 ml of distilled water. This solution was then extracted by 3x 25 ml chloroform. The chloroform layer was first dried over anhydrous $Na_2SO_4$, then filtered and evaporated to dryness by vacuum evaporator at maximum 40 °C. Dry material was finally redissolved in 5 ml of eluent and filtered by pre-injection filtration (PTFE 0,45 mm filter aid). This solution was injected (20 ml loop).

**Result:**

2,6 DMBQ content of the PABD powder is under 0.05 mg/g dry matter.
For the PABD powder produced by the method of Example 2, the measured 2,6 DMBQ content value was 0.027 mg/g dry matter.

**_Method of fingerprint chromatogram - HPLC analysis:_**

**Sample preparation**

1 g of sample was dissolved in 50 ml 96 % ethanol. This solution was then shaken for 30 min at 50 °C. The alcoholic solution was filtered, then evaporated to dryness by vacuum evaporator at maximum 50 °C. Dry material was finally redissolved in 5 ml of eluent (10 % Methanol) and filtered by pre-injection filtration (PTFE 0.45 mm filter aid). This solution was injected (20 ml loop).

**Instruments and chromatographic conditions**

| Instrument | Waters 2696 Separation Module, Alliance |
| --- | --- |
| | Waters 2996 Photodiode Array Detector |
| | Empower Pro Software, Waters |
| Column | Hypersil Gold 150x4,6; 5 u |
| Elution | Gradient* |
| | 1 ml/min |
| Detection | 290 nm |
| Mobile phase | metanol: $H_2O$ * |
| Sample weight | 1 g |

*The gradient:

| | Time | ml/min | % methanol | % water |
| --- | --- | --- | --- | --- |
| 1 | 0,01 | 1,00 | 5,0 | 95,0 |
| 2 | 20,00 | 1,00 | 30,0 | 70,0 |
| 3 | 25,00 | 1,00 | 30,0 | 70,0 |
| 4 | 30,00 | 1,00 | 5,0 | 95,0 |

Materials:

Ethanol     85% a.r. Chemolab Ltd.
Methanol    for HPLC analysis, Merck Ltd.
Bidistilled water
HPLC sample filter (PTFE 0,45 μm) Abl&E Jasco Ltd.

***Evaulation:***

Figure 1 shows the characteristic HPLC chromatogram of the PABD powder of the invention. This HPLC method detected 56 different peaks with different intensity between 0-30 min.

The most intensive peaks (with the highest area %) were:

At about 2.0 min. a peak with medium intensity (3). At about 4.5 and 4.6 min. came two intensive peaks followed quickly each other (13) and (14). After this there were detectable peaks with medium intensity at about 5.2 min., 8.0 min. and at about 14.2 min. retention time (16), (21) and (28).

The chromatogram was nearly flat between 8.0 min. and 14.0 min. There were two peaks with medium intensity at about 20.6 min. and 20.8 min., which quickly followed each other (42) and (43).

Table 1 below provides details of the peaks shown in the chromatogram.

| Nr. of peak | Name | Retention Time (min.) | Area | % Area |
|---|---|---|---|---|
| 1 | | 1,692 | 5423 | 0,05 |
| 2 | | 1,834 | 33375 | 0,32 |
| 3 | | 1,976 | 234019 | 2,26 |
| 4 | | 2,166 | 89316 | 0,86 |
| 5 | | 2,313 | 1633 | 0,02 |
| 6 | | 2,406 | 55056 | 0,53 |
| 7 | | 2,667 | 158593 | 1,53 |
| 8 | | 2,961 | 127462 | 1,23 |
| 9 | | 3,244 | 81014 | 0,78 |
| 10 | | 3,714 | 18484 | 0,18 |
| 11 | | 3,866 | 11800 | 0,11 |
| 12 | | 4,099 | 11788 | 0,11 |
| 13 | | 4,47 | 1048758 | 10,13 |
| 14 | | 4,659 | 1520027 | 14,68 |
| 15 | | 4,953 | 30368 | 0,29 |
| 16 | | 5,23 | 379480 | 3,66 |
| 17 | | 5,889 | 182271 | 1,76 |
| 18 | | 6,139 | 41522 | 0,4 |
| 19 | | 6,465 | 90560 | 0,87 |
| 20 | | 7,245 | 8170 | 0,08 |
| 21 | | 8,019 | 614460 | 5,93 |
| 22 | | 9,093 | 21077 | 0,2 |
| 23 | | 9,258 | 42690 | 0,41 |
| 24 | | 12,118 | 25684 | 0,25 |
| 25 | | 12,736 | 10727 | 0,1 |
| 26 | | 12,995 | 26523 | 0,26 |
| 27 | | 13,338 | 15323 | 0,15 |
| 28 | | 14,214 | 321872 | 3,11 |
| 29 | | 14,786 | 3001 | 0,03 |
| 30 | | 15,177 | 89833 | 0,87 |
| 31 | | 16,64 | 22906 | 0,22 |
| 32 | | 16,929 | 7755 | 0,07 |
| 33 | | 17,425 | 23967 | 0,23 |
| 34 | | 17,915 | 37534 | 0,36 |
| 35 | | 18,183 | 37277 | 0,36 |
| 36 | | 18,53 | 127798 | 1,23 |
| 37 | | 19,007 | 50679 | 0,49 |
| 38 | | 19,641 | 57096 | 0,55 |
| 39 | | 19,853 | 31796 | 0,31 |
| 40 | | 19,995 | 36416 | 0,35 |
| 41 | | 20,296 | 108337 | 1,05 |
| 42 | | 20,629 | 549427 | 5,3 |
| 43 | | 20,831 | 795858 | 7,68 |
| 44 | | 21,765 | 109628 | 1,06 |
| 45 | | 22,014 | 58490 | 0,56 |
| 46 | | 22,364 | 510739 | 4,93 |
| 47 | | 22,707 | 1867205 | 18,03 |

| | | | | |
|---|---|---|---|---|
| 48 | | 23,49 | 55823 | 0,54 |
| 49 | | 23,942 | 45909 | 0,44 |
| 50 | | 24,516 | 18143 | 0,18 |
| 51 | | 24,711 | 31814 | 0,31 |
| 52 | | 25,23 | 96306 | 0,93 |
| 53 | | 25,911 | 54406 | 0,53 |
| 54 | | 26,277 | 103264 | 1 |
| 55 | | 26,995 | 171727 | 1,66 |
| 56 | | 27,658 | 47275 | 0,46 |

Near these peaks at about 22.4 min. a peak with medium intensity (46) was detectable and immediately after this, at about 22.7 min. an intensive peak (47). After 23 min. the chromatogram showed some peaks only with very low intensity and after 28 min. there were no detected peaks.

### SDS-polyacrylamide gelelectrophoresis of PABD powder

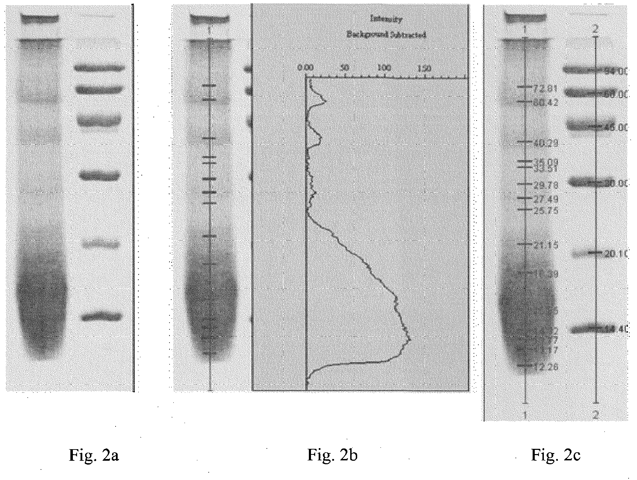

Proteins were separated from PABD powder in 15 % polyacrylamide-gel on the basis of their molecular weight (Laemmli 1970), after this protein fractions were marked by colloide-painting (Neuhoff et al. 1988.) (Figure 2a).
Electrophoregram was evaluated by Gel Doc 2000 (Bio Rad) videodensitometer (Figure 2b).
Distribution protein fractions of PABD powder was determined by compare with mixture of known proteins (Figure 2c).

Table 2 Data of distribution of protein fraction detected in PABD powder

| Lane 1 Band # | Name: #1 | | | | | |
|---|---|---|---|---|---|---|
| | Relative Front | Mol. Wt. KDa | Peak Int | Average Int | Trace Int x mm | Relative Qty |
| 1-1 | 0.135 | 72.81 | 8.53 | 4.83 | 16.366 | 0.5 |
| 1-2 | 0.174 | 60.42 | 26.16 | 12.11 | 75.690 | 2.3 |
| 1-3 | 0.285 | 40.29 | 20.23 | 9.92 | 69.774 | 2.1 |
| 1-4 | 0.337 | 35.09 | 4.18 | 2.76 | 4.309 | 0.1 |
| 1-5 | 0.354 | 33.51 | 3.19 | 2.09 | 2.722 | 0.1 |
| 1-6 | 0.399 | 29.78 | 8.64 | 5.57 | 15.962 | 0.5 |
| 1-4 | 0.438 | 27.49 | 12.54 | 8.35 | 28.284 | 0.9 |
| 1-8 | 0.469 | 25.75 | 6.78 | 4.08 | 9.571 | 0.3 |
| 1-9 | 0.563 | 21.15 | 40.40 | 20.93 | 125.378 | 3.8 |
| 1-10 | 0.642 | 18.39 | 81.38 | 62.76 | 392.246 | 11.8 |
| 1-11 | 0.743 | 15.65 | 115.83 | 103.36 | 807.476 | 24.3 |
| 1-12 | 0.799 | 14.32 x | 121.24 | 118.08 | 338.257 | 10.2 |
| 1-13 | 0.823 | 13.77 x | 126.41 | 123.98 | 129.143 | 3.9 |
| 1-14 | 0.851 | 13.17 x | 132.28 | 127.42 | 431.378 | 13.0 |
| 1-15 | 0.896 | 12.26 x | 123.20 | 49.63 | 478.213 | 14.4 |
| Molecular Weight Calculation Method: Point to Point = Known x Extrapolated | | | | | | |

Table 2 shows the relative mobility, calculated molecular weight and intensity of the different protein zones in PABD powder.

### Evaluation:

The majority of the protein fraction of PABD powder (more than 80 %) can be found between 10-22 kDa molecular weight range. At the higher molecular weight range there are only a few protein zones, with low intensity, e.g. at about 40 kDa 2.1 % and at about 60 kDa 2.3. %.

**In vivo studies**

**Examination of anti-inflammatory effect in colon-inflammatory model induced by TNBS in the case of rats.**

The 2,4,6 Trinitrobenzene-sulphonic-acid (2,4,6 TNBS) induction method represents well the pathological changes typical of patients suffering e.g. from Crohn's disease, so this model is well known and accepted in IBD-studies. The method described in Morris G.P. et al.

"Hapten-induced model of chronic inflammation and ulceration in the rat colon" Gastroenterology 1989., 96(3); 795-803 was followed.

Examinations were made in Wistar (CD) male rats. Nr: 10-10/ groups.

PABD powder was produced as described in Example 2; DH % of it's protein fraction was 20 %.

2,4,6 TNBS = 2,4,6 Trinitrobenzene-sulphonic-acid in solution.

| Name of group | Treatment |
|---|---|
| Abs. control | Untreated group |
| 2,4,6 TNBS | 2,4,6 TNBS: 10 mg/ rat |
| PABD | 2,4,6 TNBS + PADB treated group 2 g/ kg/ day p.o. treatment |
| Infliximab | 2,4,6 TINBS + infliximab 3,0 mg/ kg/day |
| SASP | 2,4,6 TNBS + Sulfasalazine 50 mg/ kg/day |

| Treatments during the study | | | | | | | | | | | | | |
|---|---|---|---|---|---|---|---|---|---|---|---|---|---|
| days | | | | | | | | | | | | | |
| -10 | -9 | -8 | -7 | -6 | -5 | -4 | -3 | -2 | -1 | 0 | 1 | 2 | 3 |
| PABD | | | | | | | | | | | | | |
| | | | | | | | | | | 2,4,6 TNBS | | | |
| infliximab | | | | infliximab | | | infliximab | | | infliximab | | | |
| | | | | | | | SASP | | | | | | |

**Evaluation of the inflammatory process**

During the method 2,4,6 TNBS (10 mg/ rat) was introduced after 12 hours starving in aether-anaesthesia into the colon. Inflammation reach the maximum on the third day. The animals were sacrificed by cervalis dislocation and the results were evaluated.

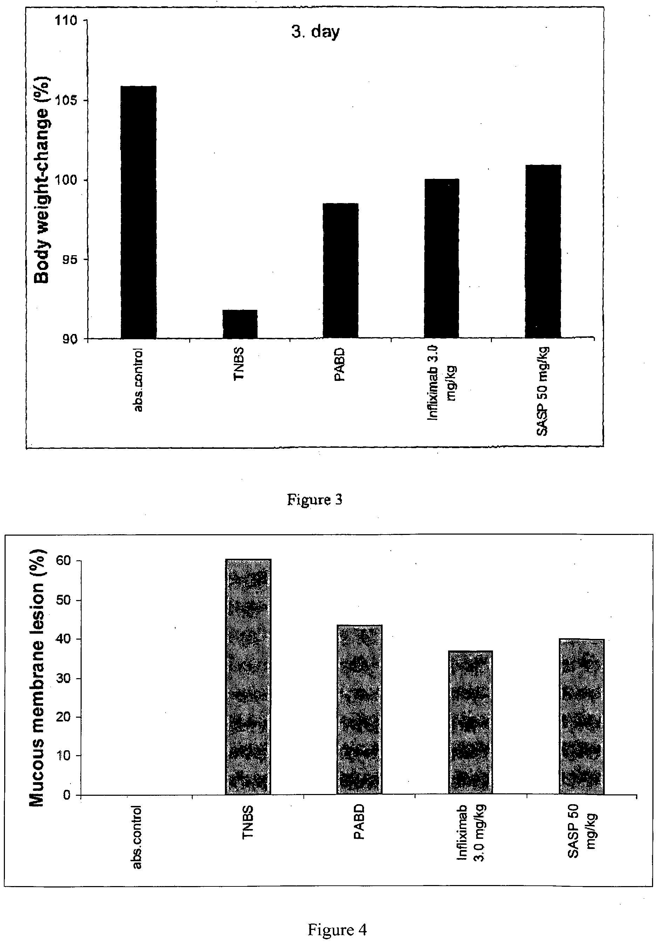

Figures 3-7 show the most important evaluation data which demonstrate the significant advantageous effects of the active agents of PABD powder in the 2,4,6 TNBS- induced inflammatory processes. The figures show the comparison with the infliximab and SASP treatments.

*Results:*

*1./ Body weight change:*

PABD powder significantly reduced the weight loss of the animals. After 2,4,6 TNBS treatment the body weight of animals decreased rapidly from 100 % to 91.8 %. PABD powder treatment could moderate the weight loss, with the body weight decreasing to 98.5 % (infliximab: 100.0 %, SASP: 100.8 %). (Figure 3).

**2./ Extension of mucous membrane lesions:**

2,4,6 TNBS treatment produced significant inflammation on the third day. This was 60.2 % expressed as the extension of the mucous membrane lesions. PABD powder treatment reduced extension of the mucous membrane lesions to 43.4 %. This value was in comparison to Infliximab treatment 36.4 %, and SASP treatment 39.7 %. (Figure 4).

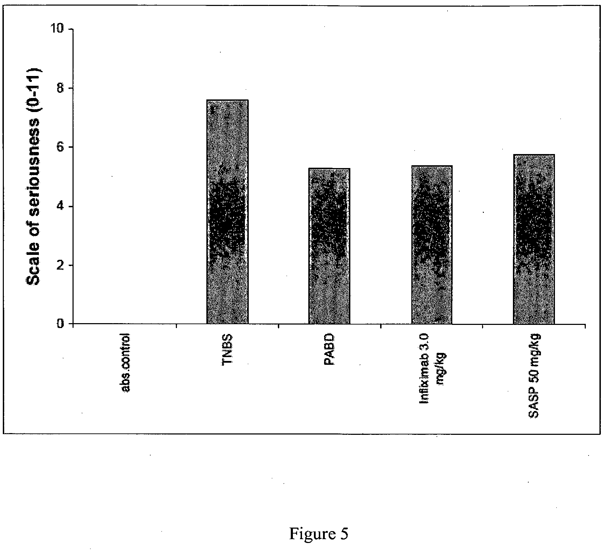

**3.I Seriousness of inflammation** was evaluated on 0-11 scale (Figure 5). Seriousness of 2,4,6 TNBS induced inflammation was 7.6 on this scale. PABD powder significantly reduced the seriousness of lesions to 5.3 on this scale. In comparision Infliximab treatment reduced the score to 5.4. and SASP treatment to 5.8.

**4.I Examination of TNF-α levels:**

Inflammatory processes cause a remarkable increase in TNF-α levels. In comparison to the control group (12.7 pg/ mg protein), in animals treated only by 2,4,6 TNBS TNF-α level reached the 420.4 pg/ mg protein value.

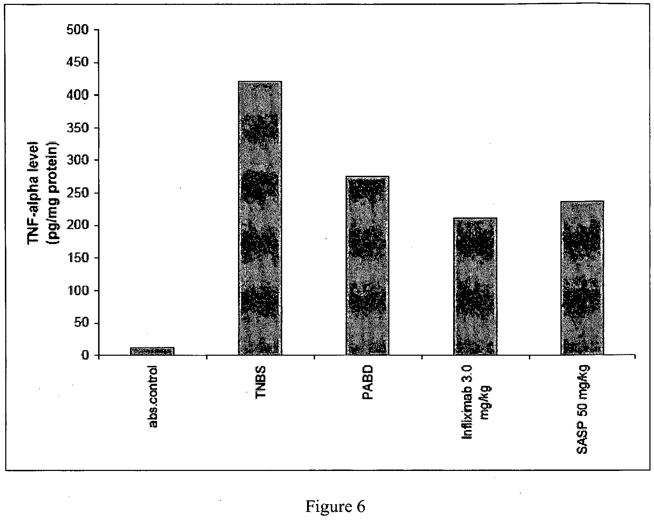

During the treatment by the new PABD powder, TNF-α level decreased significant to 275.2 pg/ mg protein level. In comparison with Infliximab treatment this value was 212.0 pg/ mg protein, and in the case of SASP treatment 236.1 pg/ mg protein. (Figure 6)

**5.I Evaluation of MPO activity:**

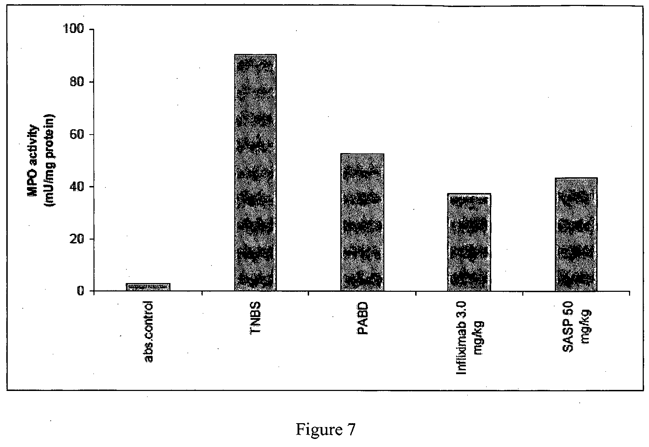

During 2,4,6 TNBS treatment MPO activity increased from 2.9 mU/mg protein to 90.4 mU/mg protein. The new PABD powder showed a significant reduction in inflammation. The PABD powder could moderate MPO activity to 52.7 mU/mg protein. (Infliximab: 37.6 mU/protein, SASP: 43.6 mU/ protein). (Figure 7)

**Summary:**

Hydrolysed, dried plant material according to the invention (PABD powder) significantly decreased the bowel inflammatory processes induced by 2,4,6 TNBS in rats. The results obtained show that the PABD powder natural origin is effective in the treatment of inflammatory diseases, a.g. in treatment of IBD.

**Claims**

1. A process for producing a dried hydrolysed wheatgerm extract comprising
   hydrolysing wheat germ with a protease enzyme in an aqueous medium, inactivating the enzyme;
   separating the residual wheat germ substrate from the hydrolysed liquid; and
   evaporating and/or drying the hydrolysed liquid.

2. A process as claimed in claim 1 wherein the degree of hydrolysis in the protein fraction of said dried material is 10-50% .

3. A process as claimed in claim 1 or claim 2 wherein the protease enzyme is pancrease powder (Pancreatis pulvis).

4. A process as claimed in any one of claims 1 to 3 wherein the hydrolysis is carried out at pH 6.5-9.5.

5. A dried hydrolysed wheatgerm extract obtainable by the process of any one of claims 1 to 4

6. The dried hydrolysed wheatgerm extract of claim 5 having an HPLC chromatogram substantially corresponding to that shown in Figure 1.

7. The dried hydrolysed wheatgerm extract of claim 5 or claim 6 for use in medicine.

8. A pharmaceutical composition comprising the dried hydrolysed wheatgerm extract of claim 5 or claim 6 and optionally one or more pharmaceutically acceptable excipients.

9. The dried hydrolysed wheatgerm extract of claim 5 or claim 6 or the pharmaceutical composition of claim 8 for use in the treatment and/or prevention of inflammatory conditions or cancer.

10. The dried hydrolysed wheatgerm extract or the pharmaceutical composition of claim 9 wherein said inflammatory condition is selected from arthritis, specifically rheumatoid arthritis, reactive arthritis and bacterial arthritis; inflammatory bowel disease, and autoimmune conditions.

11. The dried hydrolysed wheatgerm extract or the pharmaceutical composition of claim 10 wherein said inflammatory bowel disease is selected from Crohn's disease, ulcerative colitis, Collagenous colitis, Lymphocytic colitis, Ischaemic colitis, Diversion colitis, Behcet's disease and Indeterminate colitis.

**12.** A dietary supplement, or a supplementary feed comprising the dried hydrolysed wheatgerm extract of claim 5 or claim 6.

**13.** A food or feed comprising the dried hydrolysed wheatgerm extract of claim 5 or claim 6.


**Patentansprüche**

**1.** Verfahren zur Herstellung eines getrockneten, hydrolysierten Weizenkeimextrakts, umfassend:

Hydrolysieren von Weizenkeim mit einem Proteaseenzym in einem wässrigen Medium,
Inaktivieren des Enzyms;
Trennen des restlichen Weizenkeimsubstrats aus der hydrolysierten Flüssigkeit; und
Verdampfen und/oder Trocknen der hydrolysierten Flüssigkeit.

**2.** Verfahren nach Anspruch 1, wobei der Hydrolysegrad in der Proteinfraktion des getrockneten Materials 10 bis 50 % beträgt.

**3.** Verfahren nach Anspruch 1 oder Anspruch 2, wobei das Proteaseenzym Pankreas-Pulver (Pancreatis pulvis) ist.

**4.** Verfahren nach einem der Ansprüche 1 bis 3, wobei die Hydrolyse bei einem pH-Wert von 6,5 bis 9,5 durchgeführt wird.

**5.** Getrockneter, hydrolysierter Weizenkeimextrakt, der durch das Verfahren nach einem der Ansprüche 1 bis 4 gewonnen werden kann.

**6.** Getrockneter, hydrolysierter Weizenkeimextrakt nach Anspruch 5 mit einem HPLC-Chromatogramm, das im Wesentlichen dem entspricht, das in Figur 1 gezeigt wird.

**7.** Getrockneter, hydrolysierter Weizenkeimextrakt nach Anspruch 5 oder Anspruch 6 zur Verwendung in der Medizin.

**8.** Pharmazeutische Zusammensetzung, umfassend den getrockneten, hydrolysierten Weizenkeimextrakt nach Anspruch 5 oder Anspruch 6 und wahlweise einen oder mehrere pharmazeutisch verträgliche Arzneiträger.

**9.** Getrockneter, hydrolysierter Weizenkeimextrakt nach Anspruch 5 oder Anspruch 6 oder pharmazeutische Zusammensetzung nach Anspruch 8 zur Verwendung bei der Behandlung und/oder Vorbeugung von entzündlichen Zuständen oder Krebs.

**10.** Getrockneter, hydrolysierter Weizenkeimextrakt oder pharmazeutische Zusammensetzung nach Anspruch 9, wobei der entzündliche Zustand ausgewählt ist aus Arthritis, insbesondere rheumatoider Arthritis, reaktiver Arthritis und bakterieller Arthritis; entzündlicher Darmerkrankung und Autoimmunerkrankungen.

**11.** Getrockneter, hydrolysierter Weizenkeimextrakt oder pharmazeutische Zusammensetzung nach Anspruch 10, wobei die entzündliche Darmerkrankung ausgewählt ist aus Morbus Crohn, ulzerativer Kolitis, kollagener Kolitis, lymphozytärer Kolitis, ischämischer Kolitis, Diversionskolitis, Morbus Behçet und Colitis indeterminata.

**12.** Nahrungsergänzungsmittel oder Ergänzungsfutter, umfassend den getrockneten, hydrolysierten Weizenkeimextrakt nach Anspruch 5 oder Anspruch 6.

**13.** Nahrungsmittel oder Futtermittel, umfassend den getrockneten, hydrolysierten Weizenkeimextrakt nach Anspruch 5 oder Anspruch 6.


**Revendications**

**1.** Un procédé pour produire un extrait de germe de blé hydrolysé séché comprenant :

l'hydrolyse de germe de blé au moyen d'une enzyme protéase dans un milieu aqueux, l'inactivation de l'enzyme ;
la séparation du substrat de germe de blé résiduel à partir du liquide hydrolysé ; et
l'évaporation et/ou le séchage du liquide hydrolysé.

2. Un procédé tel que revendiqué dans la revendication 1, dans lequel le degré d'hydrolyse dans la fraction de protéine dudit matériau séché est de 10 à 50%.

3. Un procédé tel que revendiqué dans la revendication 1 ou la revendication 2, dans lequel l'enzyme protéase est de la poudre de pancréase (Pancreatis pulvis).

4. Un procédé tel que revendiqué dans l'une quelconque des revendications 1 à 3, dans lequel l'hydrolyse est effectuée à un pH de 6,5 à 9,5.

5. Un extrait de germe de blé hydrolysé séché capable d'être obtenu par le procédé de l'une quelconque des revendications 1 à 4.

6. L'extrait de germe de blé hydrolysé séché de la revendication 5, ayant un chromatogramme HPLC correspondant essentiellement à celui montré sur la figure 1.

7. L'extrait de germe de blé hydrolysé séché de la revendication 5 ou la revendication 6, pour utilisation en médecine.

8. Une composition pharmaceutique comprenant l'extrait de germe de blé hydrolysé séché de la revendication 5 ou la revendication 6, et le cas échéant un ou plusieurs excipients acceptables sur le plan pharmaceutique.

9. L'extrait de germe de blé hydrolysé séché de la revendication 5 ou la revendication 6 ou la composition pharmaceutique de la revendication 8 pour utilisation pour le traitement et/ou la prévention de conditions inflammatoires ou du cancer.

10. L'extrait de germe de blé hydrolysé séché ou la composition pharmaceutique de la revendication 9, dans lequel ladite condition inflammatoire est choisie parmi l'arthrite, spécifiquement l'arthrite rhumatoïde, l'arthrite réactive et l'arthrite bactérienne, la maladie inflammatoire de l'intestin, et des conditions autoimmunes.

11. L'extrait de germe de blé hydrolysé séché ou la composition pharmaceutique de la revendication 10, dans lequel ladite maladie inflammatoire de l'intestin est choisie parmi la maladie de Crohn, la colite ulcérée, la colite collagéneuse, la colite lymphocytique, la colite ischémique, la colite de diversion, la maladie de Behcet, et la colite indéterminée.

12. Un supplément diététique, ou un aliment de supplément comprenant l'extrait de germe de blé hydrolysé séché de la revendication 5 ou la revendication 6.

13. Un aliment ou une nourriture comprenant l'extrait de germe de blé hydrolysé séché de la revendication 5 ou de la revendication 6.

Figure 1

Fig. 2a

Fig. 2b

Fig. 2c

Figure 3

Figure 4

Figure 5

Figure 6

Figure 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9908694 A
- WO 2004014406 A
- WO 2004014146 A

**Non-patent literature cited in the description**

- **ADLER-NISSEN.** *J. Agric. Food Chem,* 1979, vol. 27 (6), 1256-1262